(19) Europäisches Patentamt
European Patent Office
Office européen des brevets

(11) EP 2 263 640 B1

(12) FASCICULE DE BREVET EUROPEEN

(45) Date de publication et mention de la délivrance du brevet:
**11.01.2017 Bulletin 2017/02**

(21) Numéro de dépôt: **10165698.1**

(22) Date de dépôt: **11.06.2010**

(51) Int Cl.:
***A61K 8/31*** (2006.01) ***A61K 8/58*** (2006.01)
***A61K 8/81*** (2006.01) ***A61K 8/891*** (2006.01)
***A61K 8/92*** (2006.01) ***A61Q 1/04*** (2006.01)
***A61Q 1/06*** (2006.01)

# (54) Composition cosmétique de maquillage et/ou de soin comprenant une résine tackifiante, et une association d'huiles particulières

Kosmetische Schmink- und/oder Pflegezusammensetzung, die ein klebriges Harz und eine Verbindung aus speziellen Ölen enthält

Cosmetic makeup and/or care composition comprising a tackifying resin and a combination of particular oils

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **19.06.2009 FR 0954173**

(43) Date de publication de la demande:
**22.12.2010 Bulletin 2010/51**

(73) Titulaire: **L'Oréal**
**75008 Paris (FR)**

(72) Inventeurs:
- **Ilekti, Philippe**
**94700, Maison-Alfort (FR)**
- **Guillard, Sylvie**
**77173, Chevry Cossigny (FR)**

(74) Mandataire: **Boulard, Denis**
**L'Oréal**
**Service DIPI**
**9 Rue Pierre Dreyfus**
**92110 Clichy (FR)**

(56) Documents cités:
**EP-A1- 1 604 632** **EP-A1- 1 661 549**
**EP-A1- 1 743 627** **EP-A2- 1 044 674**
**EP-A2- 1 854 451** **EP-A2- 1 944 015**
**FR-A- 933 963** **FR-A1- 2 904 218**
**FR-A1- 2 918 272** **US-A- 4 873 078**
**US-A1- 2006 134 035** **US-A1- 2007 014 745**
**US-A1- 2007 258 934**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 263 640 B1

## Description

La présente invention se rapporte à une composition cosmétique de maquillage et/ou de soin, notamment de la peau et des lèvres.

Dans le domaine cosmétique, la mise au point de formulations dotées à la fois de bonnes propriétés en termes d'application et de confort, et de propriétés satisfaisantes en termes de tenue, notamment de tenue de la brillance et de non migration, relève d'un objectif permanent.

Il est connu dans le document EP 1 405 625 des compositions de rouge à lèvres à base d'huile siliconée phénylée pour obtenir un maquillage brillant sur les lèvres. Toutefois, 2 heures après l'application sur les lèvres, l'aspect brillant s'atténue conduisant ainsi à un maquillage moins performant.

En conséquence, il demeure à ce jour un besoin d'une composition de maquillage et/ou de soin des lèvres ou de la peau, homogène et présentant une tenue améliorée de la brillance, et formant un dépôt homogène après application sur la peau ou les lèvres.

De manière inattendue, les inventeurs ont constaté que l'association d'une huile siliconée phénylée avec une résine tackifiante particulière et d'une huile volatile permet d'obtenir une composition homogène, et procurant un dépôt sur la peau ou les lèvres brillant à l'application et qui reste brillant après 2 heures.

Par ailleurs, les compositions selon l'invention sont en outre très satisfaisantes en terme de facilité d'application (glissant et délitage), de confort.

De façon plus précise, la présente invention concerne une composition cosmétique de maquillage et/ou de soin comprenant au moins une phase grasse comprenant :

- au moins une résine (également appelée « résine tackifiante ») de poids moléculaire moyen en nombre inférieur ou égal à 10000 g/mol choisie parmi les résines hydrocarbonées indéniques, et leurs mélanges,
- au moins une huile volatile, et
- au moins une huile siliconée phénylée de formule (IV) telle que définie ci-après.

L'invention a également pour objet, selon un autre aspect, un procédé cosmétique de maquillage des matières kératiniques, et notamment les lèvres, comprenant l'application sur lesdites matières kératiniques, et notamment les lèvres, d'une composition telle que définie précédemment.

L'invention a enfin pour objet l'utilisation d'une composition telle que décrite précédemment pour obtenir un maquillage de la peau ou des lèvres pour obtenir un dépôt sur la peau ou les lèvres, présentant des propriétés satisfaisantes en termes d'homogénéité du dépôt et/ou de brillance et/ou de tenue de la brillance, et/ou de tenue de la couleur.

### Résine tackifiante

La résine (également appelée résine tackifiante) utilisée dans la composition selon l'invention a un poids moléculaire moyen en nombre inférieure ou égale à 10 000 g/mol, notamment allant de 250 à 10 000 g/mol de préférence inférieure ou égale à 5 000 g/mol, notamment allant de 250 à 5 000 g/mol, mieux, inférieure ou égale à 2 000 g/mol notamment allant de 250 à 2 000 g/mol et encore mieux inférieure ou égale à 1 000 g/mol notamment allant de 250 à 1 000 g/mol.

On détermine les poids moléculaires moyens en nombre (Mn) par chromatographie liquide par perméation de gel (solvant THF, courbe d'étalonnage établie avec des étalons de polystyrène linéaire, détecteur réfractométrique).

La résine de la composition selon l'invention est avantageusement une résine dite tackifiante. De telles résines sont notamment décrites dans le Handbook of Pressure Sensitive Adhesive, edited by Donatas Satas, 3rd ed., 1989, p. 609-619.

La résine de la composition selon l'invention est choisie parmi les résines hydrocarbonées indéniques telles que les résines issues de la polymérisation en proportion majoritaire de monomère indène et en proportion minoritaire de monomère choisi parmi le styrène, le méthylindène, le méthylstyrène et leurs mélanges. Ces résines pouvant éventuellement être hydrogénées. Ces résines peuvent présenter un poids moléculaire allant de 290 à 1150 g/mol.

Comme exemples de résines indéniques, on peut citer celles commercialisées sous la référence ESCOREZ 7105 par la société Exxon Chem., NEVCHEM 100 et NEVEX 100 par la société Neville Chem., NORSOLENE S105 par la société Sartomer, PICCO 6100 par la société Hercules et RESINALL par la société Resinall Corp., ou les copolymères indène/méthylstyrène/styrène hydrogéné tels que ceux commercialisés sous la dénomination « REGALITE » par la société Eastman Chemical, en particulier REGALITE R 1100, REGALITE R 1090, REGALITE R-7100, REGALITE R1010 HYDROCARBON RESIN, REGALITE R1125 HYDROCARBON RESIN.

La résine tackifiante peut être présente dans la composition selon l'invention en une teneur allant de 0,1 à 30 % en poids, par rapport au poids total de la composition, de préférence allant de 0,3 à 20 % en poids, plus préférentiellement allant de 0,5 à 15 % en poids.

**Phase grasse liquide**

La phase grasse de la composition selon l'invention peut comprendre au moins une huile.

Par « huile », on entend un composé non aqueux, non miscible à l'eau, liquide à température ambiante (25°C) et pression atmosphérique (760 mm de Hg).

**Huile siliconée phénylée**

La composition selon l'invention comprend au moins une huile siliconée phénylée. On entend par « huile siliconée phénylée » (ou « silicone phénylée »), un organopolysiloxane comprenant au moins un groupe phényle.

L'huile siliconée phénylée est de préférence non volatile.

On entend par « huile non volatile », une huile restant sur la peau ou la fibre kératinique, plus généralement sur la matière kératinique, à température ambiante et pression atmosphérique, au moins plusieurs heures et ayant notamment une pression de vapeur inférieure à $10^{-3}$ mm de Hg (0,13 Pa). On peut également définir une huile non volatile comme ayant une vitesse d'évaporation telle que dans les conditions définies précédemment, la quantité évaporée au bout de 30 minutes est inférieure à 0,07mg/$cm^2$.

De préférence, le poids moléculaire de l'huile siliconée phénylée est compris entre 500 et 100 000 g/mol

L'huile siliconée phénylée est choisie parmi le groupe consistant en les composés de formules (I), (II), (III), (IV) et (V) suivants, la composition comprenant au moins une huile siliconée phénylée de formule (IV) :

- composés de formule (I)

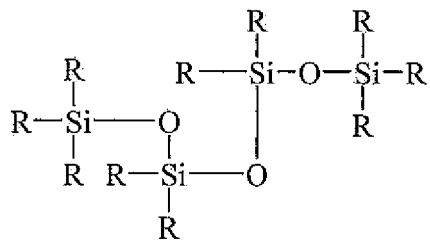

(I)

- composés de formule (II)

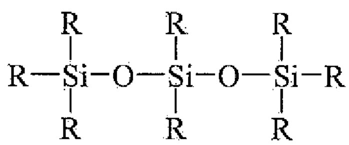

(II)

les formules (I) et (II) étant telles que les groupements R représentent indépendamment un groupe méthyle ou un groupe phényle, et qu'au moins trois des radicaux R sont des groupes phényle, voire au moins quatre, en particulier au moins cinq. On peut utiliser par exemple la triméthylpentaphényltrisiloxane (ou 1,3,5-triméthyl-1,1,3,5,5-pentaphenyl trisiloxane) vendue sous la référence (PH-1555 HRI de Dow Corning )par la société Dow Corning.

- composés de formule (III)

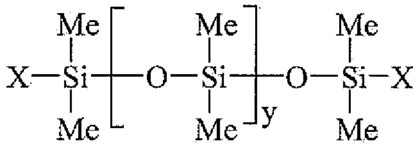

(III)

dans laquelle X représente un groupe $-CH_2-CH(CH_3)(Ph)$, Me représente un groupe méthyle et Ph un groupe phényle, et y varie entre 1 et 10 000,

- composés de formule (IV)

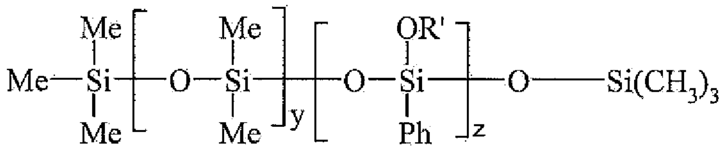

(IV)

dans laquelle Me est méthyle et Ph est phényle, OR' représente un groupe $-OSiMe_3$ et y varie entre 1 et 1000, z varie entre 1 et 1000, de telle sorte que le composé (IV) est une huile non volatile. On peut utiliser par exemple la triméthyl Siloxyphenyl Dimethicone, notamment vendue sous la référence BELSIL PDM 1000 commercialisée par la société Wacker.

- composés de formule (V)

$$[R(CH_3)_2\text{-}SiO_{1/2}]_x[SiO_{4/2}]_y \qquad (V)$$

dans laquelle R représente un groupe phénylpropyle, et x et y varient indépendamment l'un de l'autre entre 1 et 10 000, de telle sorte que x+y soit suffisamment élevée pour que le composé (V) soit une huile non volatile. On peut utiliser par exemple la phénylpropyldiméthylsiloxysilicate vendue sous la référence SilShine 151 commercialisée par GE Silicones.

Selon un mode de réalisation particulier, la composition selon l'invention contient au moins une huile siliconée phénylée de formule (IV), telle que définie ci-dessus.
L'huile siliconée phénylée de formule (II), (III), (IV) ou (V), présente de préférence un indice de réfraction supérieur à 1,4, notamment inférieur à 1,6.
Les huiles siliconées phénylées ont une viscosité choisie avantageusement dans la gamme allant de 5 à 800 000 $mm^2/s$ à 25 °C, de préférence de 10 à 500 000 $mm^2/s$, et mieux de 10 à 5 000 $mm^2/s$.
De façon préférée, l'huile siliconée phénylée est présente dans la composition selon l'invention dans une teneur allant de 0,5 à 85 % en poids, particulièrement de 5 à 70 % en poids et par exemple de 10 à 60 % en poids, par rapport au poids total de la composition.
De façon avantageuse, la composition comprend entre 10% et 85 % en poids d'huile siliconée phénylée par rapport au poids total de la composition, de préférence entre 15% et 70%, entre 15% et 60% et de façon encore préférée, entre 15% et 40%.
De façon préférée, le ratio pondéral entre l'huile siliconée phénylée, et la résine tackifiante va de 1 à 5, et de façon encore préférée de 1,5 à 4, de façon préférée de 2 à 3,5, ou encore mieux de 2,5 à 3,5.

**Huile volatile**

La composition selon l'invention comprend au moins une huile volatile.
Au sens de l'invention, une huile volatile présente à température ambiante (25 °C) et pression atmosphérique (760 mm de Hg) une pression de vapeur allant de 0,02 mm à 300 mm de Hg (2,66 Pa à 40 000 Pa) et mieux allant de 0,1 à 90 mm de Hg (13 Pa à 12 000 Pa). Les huiles non volatiles correspondent alors à une pression de vapeur inférieure à 0,02 mm de Hg (2,66 Pa), et mieux, inférieure à $10^{-3}$ mm de Hg (0, 13 Pa).
L'huile volatile peut être une huile siliconée, une huile hydrocarbonée ou une huile fluorée.

a. Huile siliconée

Selon une variante de l'invention, la phase grasse liquide comprend au moins une huile siliconée volatile.
Par « huile siliconée », on entend une huile comprenant au moins un atome de silicium, et notamment comprenant des groupes Si-O.
L'huile volatile siliconée utilisable dans l'invention peut être choisie parmi les huiles siliconées ayant un point éclair allant de 40 °C à 150 °C, de préférence ayant un point éclair supérieur à 55 °C et inférieur ou égal à 105 °C, et préférentiellement allant de 65 °C à 95 °C. Le point éclair est en particulier mesuré selon la norme iso 3679.
L'huile siliconée volatile peut être choisie parmi les huiles siliconées linéaires ou cycliques telles que les polydiméthylsiloxanes (PDMS) linéaires ou cycliques ayant de 3 à 7 atomes de silicium.
A titre d'exemple de telles huiles, on peut citer l'octyltriméthicone, l'hexyltriméthicone, la décaméthylcyclopentasiloxane (cyclopentasiloxane ou D5), l'octaméthylcyclotétrasiloxane (cyclotétradiméthylsiloxane ou D4), la dodécaméthylcyclohexasiloxane (D6), la décaméthyltétrasiloxane (L4), KF 96 A de Shin Etsu, les polydiméthysiloxanes telles que celles commercialisées sous la référence DC 200 (1,5 cSt), DC 200 (5 cSt), DC 200 (3 cSt) par Dow Corning.

b. Huile hydrocarbonée

Selon une variante de l'invention, la phase grasse liquide comprend au moins une huile hydrocarbonée volatile.

Par « huile hydrocarbonée », on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

Les huiles (également appelées solvants) hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme autres solvants (huiles) hydrocarbonés volatiles utilisables dans la composition selon l'invention, on peut également citer, les cétones liquides à température ambiante tels que méthyléthylcétone, l'acétone; les esters à chaîne courte (ayant de 3 à 8 atomes de carbone au total) tels que l'acétate d'éthyle, l'acétate de méthyle, l'acétate de propyle, l'acétate de n-butyle ; les éthers liquides à température ambiante tels que le diéthyléther, le diméthyléther ou le dichlorodiéthyléther; les alcools et notamment les monoalcools inférieurs linéaires ou ramifiés ayant de 2 à 5 atomes de carbone comme l'éthanol, l'isopropanol ou le n-propanol.

De préférence, la composition présente une teneur en huile volatile supérieure à 5% en poids, de préférence allant de 5 % à 50% en poids et allant de 10 % à 35 % en poids en poids par rapport au poids total de la composition.

Selon un mode de réalisation préféré, l'huile volatile a un point éclair supérieur à 65°c, et mieux, supérieur à 80°C. A titre d'exemple d'une telle huile volatile on peut citer l'isohexadécane.

**Huiles non volatiles**

La composition selon l'invention peut comprendre, outre l'huile volatile et l'huile siliconée phénylée, au moins une autre huile non volatile additionnelle. Celle-ci peut être en particulier choisie parmi les huiles hydrocarbonées et/ou siliconées et/ou fluorées non volatiles, et de préférence parmi les huiles hydrocarbonées.

On entend par « huile non volatile », une huile restant sur la peau ou la fibre kératinique, plus généralement sur la matière kératinique, à température ambiante et pression atmosphérique, au moins plusieurs heures et ayant notamment une pression de vapeur inférieure à $10^{-3}$ mm de Hg (0,13 Pa). On peut également définir une huile non volatile comme ayant une vitesse d'évaporation telle que dans les conditions définies précédemment, la quantité évaporée au bout de 30 minutes est inférieure à 0,07mg/$cm^2$.

Ces huiles peuvent être d'origine végétale, minérale ou synthétique.

Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces derniers pouvant être linéaires ou ramifiées, saturées ou insaturées, comme les triglycérides des acides heptanoïque, octanoïque ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations de «Miglyol 810® », « 812® » et « 818® » par la société Dynamit Nobel,
- les éthers de synthèse;
- les hydrocarbures linéaires ou ramifiés, d'origine minérale ou synthétique tels que l'huile de paraffine ou ses dérivés, la vaseline, le polyisobutène hydrogéné tel que le Parléam® commercialisé par la société NIPPON OIL FATS, le squalane, les polybutylènes tels que L'INDOPOL H-100 (de masse molaire ou MW=965 g/mol), L'INDOPOL H-300 (MW=1340 g/mol), L'INDOPOL H-1500 (MW=2160g/mol) commercialisés ou fabriqués par la société AMOCO, les polyisobutylènes hydrogénés tels que le PANALANE H-300 E commercialisés ou fabriqué par la société AMOCO (MW =1340 g/mol), le VISEAL 20000 commercialisé ou fabriqué par la société SYNTEAL (MW=6000 g/mol), le

REWOPAL PIB 1000 commercialisé ou fabriqué par la société WITCO (MW=1000 g/mol), les polydécènes et les polydécènes hydrogénés tels que : le PURESYN 10 (MW=723 g/mol), le PURESYN 150 (MW=9200 g/mol) commercialisé ou fabriqués par la société MOBIL CHEMICALS, et leurs mélanges;

- les esters d'acide gras, en particulier de 4 à 22 atomes de carbone, et notamment d'acide octanoïque, d'acide heptanoïque, d'acide lanolique, d'acide oléique, d'acide laurique, d'acide stéarique comme le dioctanoate de propylène glycol, le monoisostéarate de propylène glycol, le poly-glycéryl 2-diisostéarate, le diheptanoate de néopentylglycol,
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 11$, comme par exemple l'huile de Purcellin (octanoate de cétostéaryle), l'isononanoate d'isononyle, le benzoate d'alcool en $C_{12}$ à $C_{15}$, le palmitate d'éthyl 2-hexyle, le stéarate d'octyl-2 dodécyle, l'érucate d'octyl-2 dodécyle, l'isostéarate d'isostéaryle, le benzoate d'octyl-2 dodécyle, des octanoates, décanoates ou ricinoléates d'alcools ou de polyalcools, le myristate d'isopropyle, le palmitate d'isopropyle, le stéarate de butyle, le laurate d'hexyle, le palmitate de 2-éthyl-hexyle, le laurate de 2-hexyl-décyle, le palmitate de 2-octyl-décyle, le myristate de 2-octyldodécyle, le néopentanoate d'isodécyle ;
- les esters hydroxylés comme le lactate d'isostéaryle, l'octylhydroxystéarate, l'hydroxystéarate d'octyldodécyle, le diisostéarylmalate, le citrate de triisocétyle, le triisostéarate de glycérine ou de diglycérine; le diisononanoate de diéthylèneglycol ; et
- les esters du pentaérythritol comme le tétra décyl -2 tétradécanoate de pentaérythrityle (MW=1538 g/mol), le tétraisostéarate de pentaérythrityle (MW=1202 g/mol), le tétraisononanoate de pentaérythrityle (MW=697 g/mol),
- les esters d'acides aromatiques et d'alcools comprenant 4 à 22 atomes de carbone, notamment le trimellitate de tridécyle,
- un polyester résultant de l'estérification d'au moins un triglycéride d'acide(s) carboxylique(s) hydroxylé(s) par un acide monocarboxylique aliphatique et par un acide dicarboxylique aliphatique, éventuellement insaturé comme l'huile de ricin d'acide succinique et d'acide isostéarique commercialisée sous la référence Zénigloss par Zénitech,
- les esters de dimère diol et de dimère diacide de formule générale $HO-R^1-(-OCO-R^2-COO-R^1-)_h-OH$, dans laquelle :

  $R^1$ représente un reste de dimère diol obtenu par hydrogénation du diacide dilinoléique
  $R^2$ représente un reste de diacide dilinoléique hydrogéné, et
  h représente un entier variant de 1 à 9,

  notamment les esters de diacides dilinoléiques et de dimères diols dilinoléiques commercialisés par la société NIPPON FINE CHEMICAL sous la dénomination commerciale LUSPLAN DD-DA5® et DD-DA7®,
- les copolymères de vinylpyrrolidone/1-héxadécène, par exemple commercialisé sous la dénomination ANTARON V-216 par la société ISP (MW=7300 g/mol),
- les alcools gras liquides à température ambiante à chaîne carbonée ramifiée et/ou insaturée ayant de 8 à 26 atomes de carbone comme l'alcool oléique, l'alcool linoléique ou linolénique, l'alcool isostéarique ou l'octyl dodécanol; les acides gras supérieurs en $C_8$-$C_{26}$ tels que l'acide oléique, l'acide linoléique, l'acide linolénique, ou l'acide isostéarique ;
- et leurs mélanges.

Les huiles de silicone non volatiles utilisables dans la composition selon l'invention, différentes de l'huile siliconée phénylée, peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone.
De façon préférée, la composition contient au moins une huile hydrocarbonée non volatile choisie parmi les copolymères vinylpyrrolidone/1-héxadécène, ANTARON V-216 (également appelé Ganex V216) commercialisé ou fabriqué par la société ISP (MW=7300 g/mol),
**[0049]** De façon préférée, l'huile non volatile additionnelle (différente de l'huile siliconée phénylée) peut être présente en une teneur allant de 0,1 à 60 % en poids, notamment allant de 0,5 à 50 % en poids, et en particulier allant de 1 à 40 % en poids, par rapport au poids total de la composition.
Outre les huiles décrites précédemment, la phase grasse peut également comprendre au moins un corps gras qui n'est pas liquide à température ambiante (25°C) et à pression atmosphérique, appelé corps gras solide, choisi parmi les cires et les corps gras pâteux.

**Cire(s)**

La cire considérée dans le cadre de la présente invention est d'une manière générale un composé lipophile,

solide à température ambiante (25 °C), à changement d'état solide/liquide réversible, ayant un point de fusion supérieur ou égal à 30 °C pouvant aller jusqu'à 200 °C et notamment jusqu'à 120 °C.

En particulier, les cires convenant à l'invention peuvent présenter un point de fusion supérieur ou égal à 45 °C, et en particulier supérieur ou égal à 55 °C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, à température ambiante d'origine animale, végétale, minérale ou de synthèse et leurs mélanges.

A titre illustratif des cires convenant à l'invention, on peut notamment citer les cires hydrocarbonées comme la cire d'abeille, la cire de lanoline, et les cires d'insectes de Chine, la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires de silicone ($C_{30-45}$ ALKYL DIMETHICONE), les cires fluorées.

On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A- 2792190.

Comme cire, on peut utiliser un (hydroxystéaryloxy)stéarate d'alkyle en $C_{20}$-$C_{40}$ (le groupe alkyle comprenant de 20 à 40 atomes de carbone), seul ou en mélange.

Une telle cire est notamment vendue sous les dénominations « Kester Wax K 82 P® », « Hydroxypolyester K 82 P® » et « Kester Wax K 80 P® » par la société KOSTER KEUNEN.

Comme micro cires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 250S® par la société MICRO POWDERS et les micro cires de polytétrafluoroéthylène telles que celles commercialisées sous les dénominations de Microslip 519® et 519 L® par la société MICRO POWDERS.

Selon un mode de réalisation préféré de l'invention, la composition comprend au moins une cire.

Avantageusement, la cire est choisie parmi les cires hydrocarbonées, de façon préférée parmi les cires de polyéthylène.

La composition selon l'invention peut comprendre une teneur en cires allant de 0,1 à 30 % en poids par rapport au poids total de la composition, en particulier elle peut en contenir de 0,5 à 20 %, plus particulièrement de 1 à 15 %.

Selon un autre mode de réalisation, la composition selon l'invention est exempte de cire. Selon ce mode de réalisation, la composition est donc de préférence sous forme liquide.

Selon un mode de réalisation préféré, la composition selon l'invention comprend au moins une résine hydrocarbonée (de préférence choisie parmi les copolymères indène/méthylstyrène/styrène hydrogéné tel que ceux commercialisés sous la dénomination « Régalite » par la société Eastman Chemical , une huile siliconée phénylée non volatile, une huile volatile, une huile non volatile additionnelle, de préférence choisie parmi les copolymères vinylpyrrolidone/1-hexadécène et une cire de polyéthylène.

**Pâteux**

La composition selon l'invention peut comprendre, outre les cires, un autre corps gras solide tel qu'au moins un composé pâteux.

Par "pâteux" au sens de la présente invention, on entend un composé gras lipophile à changement d'état solide/liquide réversible, présentant à l'état solide une organisation cristalline anisotrope, et comportant à la température de 23°C une fraction liquide et une fraction solide.

En d'autres termes, la température de fusion commençante du composé pâteux peut être inférieure à 23°C. La fraction liquide du composé pâteux mesurée à 23°C peut représenter 9 à 97% en poids du composé. Cette fraction liquide à 23°C représente de préférence entre 15 et 85%, de préférence encore entre 40 et 85% en poids.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 50 à 100%, de préférence encore de 60 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux est de préférence choisi parmi les composés synthétiques et les composés d'origine végétale. Un composé pâteux peut être obtenu par synthèse à partir de produits de départ d'origine végétale.

Le composé pâteux peut avantageusement choisi parmi :

- la lanoline et ses dérivés
- les composés siliconés polymères ou non
- les composés fluorés polymères ou non
- les polymères vinyliques, notamment :
  - les homopolymères et copolymères d'oléfines ;
  - les homopolymères et copolymères de diènes hydrogénés ;
  - les oligomères linéaires ou ramifiés, homo ou copolymères de (méth)acrylates d'alkyles ayant de préférence un groupement alkyle en $C_8$-$C_{30}$;
  - les oligomères homo et copolymères d'esters vinyliques ayant des groupements alkyles en $C_8$-$C_{30}$;
  - les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$,
- les esters,
- et leurs mélanges.

Parmi les esters, on préfère notamment :

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,

- le propionate d'arachidyle commercialisé sous la marque Waxenol 801 par Alzo,
- les esters de phytostérol,
- les triglycérides d'acides gras et leurs dérivés,
- les esters de pentaérythritol,
- les polyesters non réticulés résultant de la polycondensation entre un acide dicarboxylique ou un polyacide carboxylique linéaire ou ramifié en $C_4$-$C_{50}$ et un diol ou un polyol en $C_2$-$C_{50}$,
- les esters aliphatiques d'ester résultant de l'estérification d'un ester d'acide hydroxycarboxylique aliphatique par un acide carboxylique aliphatique,
- les polyesters résultant de l'estérification, par un acide polycarboxylique, d'un ester d'acide hydroxy carboxylique aliphatique, ledit ester comprenant au moins deux groupes hydroxyle tels que les produits Risocast DA-H®, et Risocast DA-L®,
- les esters de dimère diol et dimère diacide, le cas échéant, estérifiés sur leur(s) fonction(s) alcool(s) ou acide(s) libre(s) par des radicaux acides ou alcools tels que le Plandool-G,
- et leurs mélanges.

Parmi les composés pâteux d'origine végétale, on choisira de préférence un mélange de stérols de soja et de pentaérythritol oxyéthyléné (50E) oxypropyléné (5 OP), commercialisé sous la référence Lanolide par la société VEVY.
Selon un premier mode de réalisation, la composition comprend une teneur totale en corps gras pâteux allant de 0,5 à 50% en poids par rapport au poids de la composition, de préférence de 1 à 40% ou encore mieux, de 5 à 30%.
Selon un autre mode de réalisation mode de réalisation, la composition comprend moins de 10 % en poids, de préférence moins de 7 %, mieux moins de 5 %, et encore mieux moins de 3 % en poids de corps gras pâteux par rapport au poids total de la composition. De préférence encore, la composition est totalement exempte de corps gras pâteux.

## Charge

Avantageusement, la composition selon l'invention comprend au moins une charge, notamment en une teneur totale allant de 0,01 % à 50 % en poids, par rapport au poids total de la composition, de préférence allant de 0,01 % à 30 % en poids.
Par charges, il faut comprendre au sens de la présente invention, des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-ß-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la Société Dow Corning) et les microbilles de résine de silicone (Tospearls® de Toshiba, par exemple), les poudres de polyuréthane (par exemple PLASTIC POWDER D-400® ou PLASTIC POWDER D-800® de la société TOSHIKI), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

## Agent structurant/épaississant

La composition selon l'invention peut comprendre, outre les cires éventuellement présentes, au moins un agent structurant choisi parmi les gélifiants lipophiles et leurs mélanges.

## Gélifiants lipophiles

Selon un mode de réalisation, la composition selon l'invention peut comprendre au moins un gélifiant. Les gélifiants utilisables dans les compositions selon l'invention peuvent être des gélifiants lipophiles organiques ou minéraux, polymériques ou moléculaires.
Comme gélifiant lipophile minéral, on peut citer les argiles éventuellement modifiées comme les hectorites modifiées par un chlorure d'ammonium en $C_{10}$ à $C_{22}$, comme l'hectorite modifiée par du chlorure de di-stéaryl di-méthyl ammonium

telle que, par exemple, celle commercialisée sous la dénomination de Bentone 38V® par la société ELEMENTIS.

On peut également citer la silice pyrogénée éventuellement traitée hydrophobe en surface dont la taille des particules est inférieure à 1 μm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :

- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (8ème édition, 2000). Elles sont par exemple commercialisées sous les références Aerosil R812® par la société DEGUSSA, CAB-O-SIL TS-530® par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (8ème édition, 2000). Elles sont par exemple commercialisées sous les références Aerosil R972®, et Aerosil R974® par la société DEGUSSA, CAB-O-SIL TS-610® et CAB-O-SIL TS-720® par la société CABOT.

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR.

On peut également utiliser les polyamides siliconés du type polyorganosiloxane tels que ceux décrits dans les documents US-A-5,874,069, US-A-5,919,441, US-A-6,051,216 et US-A-5,981,680.

Ces polymères siliconés peuvent appartenir aux deux familles suivantes :

- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

**Matières colorantes**

La composition cosmétique selon l'invention peut contenir un agent de coloration (également appelé matière colorante) qui peut être choisi parmi les colorants hydrosolubles ou liposolubles, les pigments, les nacres et leurs mélanges.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules blanches ou colorées, minérales ou organiques, insolubles dans une solution aqueuse, destinées à colorer et/ou opacifier le film résultant.

Les pigments peuvent être présents à raison de 0,01 à 20 % en poids, notamment de 0,01 à 15 % en poids, et en particulier de 0,02 à 10 % en poids, par rapport au poids total de la composition cosmétique.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica titane recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA, FLAMENCO et DUOCHROME (sur base de mica) commercialisées par la société ENGELHARD, les nacres TIMIRON commercialisées par la société MERCK, les nacres sur base de mica PRESTIGE commercialisées par la société ECKART et les nacres sur base de mica synthétique SUNSHINE commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Par « colorants », il faut comprendre des composés généralement organiques solubles dans les corps gras comme les huiles ou dans une phase hydroalcoolique.

La composition cosmétique selon l'invention peut comprendre également des colorants hydrosolubles ou liposolubles. Les colorants liposolubles sont par exemple le rouge Soudan, le DC Red 17, le DC Green 6, le β-carotène, le brun Soudan, le DC Yellow 11, le DC Violet 2, le DC orange 5, le jaune quinoléine. Les colorants hydrosolubles sont par exemple le jus de betterave, le bleu de méthylène.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques. Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :

- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni,

Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures

A titre illustratif de ces particules, on peut citer des particules d'aluminium, telles que celles commercialisées sous les dénominations STARBRITE 1200 EAC® par la société SIBERLINE et METALURE® par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique comme des poudres de bronze (alliage cuivre et zinc) enrobé de silice commercialisées sous la dénomination de Visionaire Bright Natural Gold de la société Eckart.

Il peut encore s'agir de particules comportant un substrat de verre comme celles commercialisées par la société NIPPON SHEET GLASS sous les dénominations MICROGLASS METASHINE.

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : $Al/SiO_2/Al/SiO_2/Al$, des pigments ayant cette structure étant commercialisés par la société DUPONT DE NEMOURS ; $Cr/MgF_2/Al/MgF_2/Cr$, des pigments ayant cette structure étant commercialisés sous la dénomination CHROMAFLAIR par la société FLEX ; $MoS_2/SiO_2/Al/SiO_2/MoS_2$ ; $Fe_2O_3/SiO_2/Al/SiO_2/Fe_2O_3$, et $Fe_2O_3/SiO_2/Fe_2O_3/SiO_2/Fe_2O_3$, des pigments ayant ces structures étant commercialisés sous la dénomination SICOPEARL par la société BASF ; $MoS_2/SiO_2$/mica-oxyde/$SiO_2/MoS_2$ ; $Fe_2O_3/SiO_2$/mica-oxyde/$SiO_2/Fe_2O_3$ ; $TiO_2/SiO_2/TiO_2$ et $TiO_2/Al_2O_3/TiO_2$; SnO/TiO2/SiO2/TiO2/SnO ; $Fe_2O_3/SiO_2/Fe_2O_3$ ; SnO/mica/$TiO_2/SiO_2/TiO_2$/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure $Fe_2O_3/SiO_2/Al/ SiO_2/Fe_2O_3$ on passe du doré-vert au gris-rouge pour des couches de $SiO_2$ de 320 à 350 nm ; du rouge au doré pour des couches de $SiO_2$ de 380 à 400 nm ; du violet au vert pour des couches de $SiO_2$ de 410 à 420 nm ; du cuivre au rouge pour des couches de $SiO_2$ de 430 à 440 nm.

On peut citer, à titre d'exemple de pigments à structure multicouche polymérique, ceux commercialisés par la société 3M sous la dénomination COLOR GLITTER.

Comme particules goniochromatiques à cristaux liquides, on peut utiliser par exemple celles vendues par la société CHENIX ainsi que celle commercialisées sous la dénomination HELICONE® HC par la société WACKER.

**Ingrédients cosmétiques usuels additionnels**

La composition selon l'invention peut comprendre en outre tout ingrédient cosmétique usuel pouvant être choisi notamment parmi les polymères filmogènes, les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les agents déodorants, les sequestrants, les agents filmogènes, les polymères semi-cristallins et leurs mélanges.

Bien entendu, l'homme du métier veillera à choisir les éventuels ingrédients complémentaires et/ou leur quantité de telle manière que les propriétés avantageuses de la composition selon l'invention ne soient pas ou substantiellement pas altérées par l'adjonction envisagée.

**Protocole de mesure de la dureté :**

La mesure est réalisée selon le protocole suivant :

Le stick de rouge à lèvres est conservé à 20 °C pendant 24 heures avant la mesure de la dureté.

La dureté peut être mesurée à 20 °C par la méthode dite « du fil à couper le beurre », qui consiste à couper transversalement un bâton de produit, de préférence cylindrique de révolution, à l'aide d'un fil rigide de tungstène de diamètre 250 $\mu$m en déplaçant le fil relativement au stick à une vitesse de 100 mm/min.

La dureté des échantillons de compositions de l'invention, exprimée en $Nm^{-1}$, est mesurée au moyen d'un dynamomètre DFGS2 commercialisé par la société INDELCO-CHATILLON.

La mesure est reproduite trois fois puis moyennée. La moyenne des trois valeurs lues au moyen du dynamomètre mentionné ci-dessus, notée Y, est donnée en grammes. Cette moyenne est convertie en Newton puis divisée par L qui représente la dimension la plus élevée traversée par le fil. Dans le cas d'un bâton cylindrique, L est égal au diamètre (en mètres).

La dureté est convertie en $Nm^{-1}$ par l'équation ci-dessous :

$$(Yx\ 10^{-3}\ x9.8)/L$$

Pour une mesure à une température différente, on conserve le stick 24 heures à cette nouvelle température avant la mesure.

Selon cette méthode, la dureté à 20 °C d'exemples de composition selon un aspect de l'invention est supérieure à 30 $Nm^{-1}$ notamment supérieure à 40 $Nm^{-1}$ de préférence supérieure à 50 $Nm^{-1}$.

Selon cette méthode, la dureté à 20 °C d'exemples de composition selon un aspect de l'invention est inférieure à 500 $Nm^{-1}$ notamment inférieure à 400 $Nm^{-1}$ de préférence inférieure à 300 $Nm^{-1}$.

En particulier, par « composition solide » on entend une composition dont la dureté est supérieure à 30 $Nm^{-1}$, de préférence supérieure à 50 $Nm^{-1}$.

De façon préférée, la composition selon l'invention est solide.

Le terme « solide » caractérise l'état de la composition à température ambiante (20°C) et à pression atmosphérique (760 mm de Hg).

De façon avantageuse, la composition selon l'invention se présente sous la forme de fond de teint solide, de bâton ou pâte de rouge à lèvres, de produit anti-cernes, ou contours des yeux, d'eye-liner, de mascara, d'ombre à paupières, de produit de maquillage du corps ou encore un produit de coloration de la peau.

En particulier, la composition de l'invention peut se présenter sous la forme d'un produit coloré de maquillage des lèvres comme un rouge à lèvres, un brillant à lèvres ou un crayon, présentant éventuellement des propriétés de soin ou de traitement.

De façon préférée, la composition selon l'invention comprend moins de 3%, ou mieux, moins de 1% d'eau en poids par rapport au poids total de la composition. De façon encore préférée la composition est totalement anhydre. Par anhydre, on entend notamment que l'eau n'est de préférence pas ajoutée délibérément dans la composition mais peut être présente à l'état de trace dans les différents composés utilisés dans la composition.

Selon un mode de réalisation préféré, la composition selon l'invention se présente sous la forme d'un stick anhydre.

L'invention est illustrée plus en détail dans les exemples suivants donnés à titre illustratif et sans caractère limitatif. Les pourcentages sont des pourcentages en poids.

<u>**Exemples 1 et 2** : **Rouges à lèvres en stick**</u>

On a préparé une composition 1 de rouge à lèvres selon l'invention et une composition 2 comparative hors l'invention ne contenant pas de résine tackifiante, comprenant les ingrédients suivants (quantités en pourcentage massique) :

| | **Matières premières (Nom INCI US)** | **Composition 1 selon l'invention** | **Composition 2 comparative (hors invention)** |
|---|---|---|---|
| Phase A | NEOPENTANOATE D'OCTYLDODECYL | 13,00 | 14,24 |
| | POLYISOBUTENE HYDROGENE (PARLEAM de chez NOF Corporation) | 6,00 | 6,83 |
| | COPOLYMERE VINYLPYRROLIDONE/HEXAD ECENE (Antaron V216 de chez ISP) | 9,00 | 9,89 |
| Phase B | ISOHEXADECANE | 20,00 | 21,96 |
| | COPOLYMERE STYRENE/METHYL STYRENE/INDENE HYDROGENE (REGALITE R1100 de EASTMAN CHEMICAL) | 8,00 | - |

(suite)

| | Matières premières (Nom INCI US) | Composition 1 selon l'invention | Composition 2 comparative (hors invention) |
|---|---|---|---|
| Phase C | COPOLYMERE VINYLPYRROLIDONE/EICOSE NE (Antaron V220F de chez ISP) | 2,00 | 1,98 |
| | CIRE de POLYETHYLENE (Performalene 500-L de New Phase Technologies) | 11,00 | 11,97 |
| Phase D | Oxydes de fer | 3,43 | 3,39 |
| | Colorant bleu | 1,05 | 1,04 |
| | Dioxyde de titane | 1,43 | 1,41 |
| | Colorant Rouge | 2,08 | 2,06 |
| Phase E | TRIMETHYLSILOXYPHENYL DIMETHICONE (BELSIL 1000 de chez Wacker) | 23 | 25,23 |
| | Total : | 100 | 100 |

Les compositions 1 et 2 sont obtenues selon le protocole suivant :

Dans un premier temps, les charges et les pigments de la phase D sont broyés dans une partie de la phase huileuse A.

Le reste des ingrédients liposolubles sont ensuite mélangés à une température de l'ordre de 100 °C. Le broyat est alors ajouté dans la phase huileuse.

Enfin, la composition est coulée dans un moule permettant l'obtention de sticks de 12,7 mm de diamètre et le tout est laissé refroidir dans un congélateur environ une heure.

La dureté de la composition 1 est de 104 $Nm^{-1}$ et la dureté de la composition comparative 2 est de 103 $Nm^{-1}$. Les deux compositions ont donc une dureté similaire.

2 heures après application de chaque composition sur les lèvres, on a observé que le dépôt sur les lèvres réalisé avec la composition 1 est plus brillant et plus confortable (pas de tiraillement ni de dessèchement des lèvres), que celui réalisé avec la composition 2 comparative.

Ainsi, la présence de la résine Régalite R 1100 premet d'améliorer la tenue de la brillance et le confort du produit de maquillage.

## Revendications

1. Composition cosmétique de maquillage et/ou de soin comprenant au moins une phase grasse comprenant :

   - au moins une résine de poids moléculaire moyen en nombre inférieur ou égal à 10000 g/mol choisie parmi les résines hydrocarbonées indéniques, et leurs mélanges,
   - au moins une huile volatile, et

   au moins une huile siliconée phénylée de formule (IV) :

   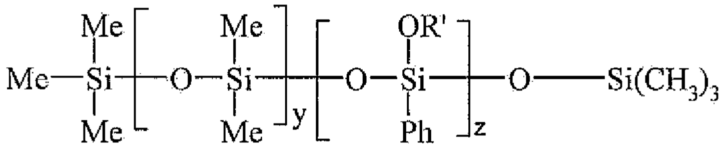

   (IV)

   dans laquelle Me représente un groupe méthyle et Ph représente un groupe phényle, OR' représente un groupe $-OSiMe_3$ et y varie entre 1 et 1000, z varie entre 1 et 1000.

2. Composition selon la revendication précédente, **caractérisée en ce que** la résine hydrocarbonée indénique est

issue de la polymérisation de monomère indène et de monomère choisi parmi le styrène, le méthylindène, le méthylstyrène et leurs mélanges.

**3.** Composition selon l'une des revendications précédentes, **caractérisée en ce que** la résine hydrocarbonée indénique est hydrogénée.

**4.** Composition selon l'une quelconque des revendications 1 à 3, **caractérisée en ce que** la résine est une résine indénique choisie parmi les copolymères indène/méthylstyrène/styrène hydrogénés.

**5.** Composition selon l'une quelconque des revendications précédentes, **caractérisée en ce que** la résine est présente en une teneur allant de 0,1 à 30 % en poids, par rapport au poids total de la composition, de préférence allant de 0,3 à 20 % en poids, plus préférentiellement allant de 0,5 à 15% en poids.

**6.** Composition selon l'une quelconque des revendications précédentes, **caractérisée en ce que** l'huile siliconée phénylée est présente en une teneur allant de 0,5 à 85 % en poids, particulièrement de 5 à 70 % en poids, par rapport au poids total de la composition.

**7.** Composition selon l'une quelconque des revendications précédentes, **caractérisée en ce qu'**elle présente un ratio pondéral entre ladite huile siliconée phénylée et ladite résine va de 1 à 5, et de façon encore préférée de 1,5 à 4, de façon préférée de 2 à 3,5, ou encore mieux de 2,5 à 3,5.

**8.** Composition selon l'une quelconque des revendications précédentes, **caractérisée en ce que** ladite huile volatile est une huile hydrocarbonée choisie parmi les huiles hydrocarbonées ayant de 8 à 16 atomes de carbones, de préférence, l'huile volatile a un point éclair supérieur à 80°C, telle que l'isohexadécane.

**9.** Composition selon l'une quelconque des revendications précédentes, **caractérisée en ce qu'**elle présente une teneur en huile volatile allant de 5 % à 50% en poids et allant de 10 % à 35 % en poids en poids par rapport au poids total de la composition.

**10.** Composition selon l'une quelconque des revendications précédentes, **caractérisée en ce qu'**elle comprend au moins une huile non volatile hydrocarbonée additionnelle.

**11.** Composition selon la revendication précédente, **caractérisée en ce que** l'huile non volatile hydrocarbonée additionnelle est choisie parmi les copolymères vinylpyrrolidone/1-héxadécène.

**12.** Composition selon la revendication précédente, **caractérisée en ce que** ladite huile non volatile additionnelle est présente en une teneur allant de 0,1 à 60 % en poids, notamment allant de 0,5 à 50 % en poids, et en particulier allant de 1 à 40 % en poids, par rapport au poids total de ladite composition.

**13.** Composition selon l'une quelconque des revendications précédentes, **caractérisée en ce qu'**elle comprend de 0,1 à 30 % en poids de cire, par rapport au poids total de la composition.

**14.** Composition selon l'une quelconque des revendications 1 à 12, **caractérisée en ce qu'**elle est exempte de cire.

**15.** Composition selon l'une quelconque des revendications précédentes, **caractérisée en ce qu'**elle comprend au moins une charge, et/ou au moins un corps gras pâteux, et/ou au moins une matière colorante.

**16.** Composition selon la revendication précédente, **caractérisée en ce qu'**elle comprend moins de 3%, ou mieux, moins de 1% d'eau en poids par rapport au poids total de la composition, voire est totalement anhydre.

**17.** Composition selon l'une quelconque des revendications précédentes, **caractérisée en ce que** ladite composition est sous forme solide.

**18.** Procédé de maquillage ou de soin de la peau et/ou des lèvres, dans lequel on applique sur la peau et/ou les lèvres une composition telle que définie selon l'une quelconque des revendications précédentes.

**19.** Utilisation d'une composition telle que définie selon l'une quelconque des revendications 1 à 17, pour obtenir un maquillage de la peau et/ou des lèvres pour obtenir un dépôt sur la peau et/ou les lèvres, présentant des propriétés

satisfaisantes en termes d'homogénéité du dépôt et/ou de brillance et/ou de tenue de la brillance, et/ou de tenue de la couleur

## Patentansprüche

1. Kosmetische Schmink- und/oder Pflegezusammensetzung, umfassend mindestens eine Fettphase, die Folgendes umfasst:

    - mindestens ein Harz mit einer zahlengemittelten Molekülmasse von weniger als oder gleich 10000 g/mol, ausgewählt aus Inden-Kohlenwasserstoffharzen und ihren Gemischen,
    - mindestens ein flüchtiges Öl und
    - mindestens ein phenyliertes Silikonöl der Formel (IV):

    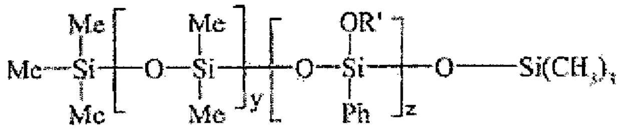

    (IV) ,

    worin Me für eine Methylgruppe steht und Ph für eine Phenylgruppe steht, OR' für eine Gruppe $-OSiMe_3$ steht und y zwischen 1 und 1000 variiert, z zwischen 1 und 1000 variiert.

2. Zusammensetzung nach dem vorhergehenden Anspruch, **dadurch gekennzeichnet, dass** der Inden-Kohlenwasserstoff Harz aus der Polymerisation von Inden-Monomer und Monomer, das aus Styrol, Methylinden, Methylstyrol und ihren Gemischen ausgewählt ist, stammt.

3. Zusammensetzung nach einem der vorhergehenden Ansprüche, **dadurch gekennzeichnet, dass** das Inden-Kohlenwasserstoffharz hydriert ist.

4. Zusammensetzung nach einem der Ansprüche 1 bis 3, **dadurch gekennzeichnet, dass** es sich bei dem Harz um ein Inden-Harz handelt, das aus hydrierten Inden/Methylstyrol/Styrol-Copolymeren ausgewählt ist.

5. Zusammensetzung nach einem der vorhergehenden Ansprüche, **dadurch gekennzeichnet, dass** das Harz in einem Gehalt von 0,1 bis 30 Gew.-%, bezogen auf das Gesamtgewicht der Zusammensetzung, vorzugsweise von 0,3 bis 20 Gew.-%, noch stärker bevorzugt von 0,5 bis 15 New.-%, vorhanden ist.

6. Zusammensetzung nach einem der vorhergehenden Ansprüche, **dadurch gekennzeichnet, dass** das phenylierte Silikonöl in einem Gehalt von 0,5 bis 85 Gew.-%, insbesondere von 5 bis 70 Gew.-%, bezogen auf das Gesamtgewicht der Zusammensetzung, vorhanden ist.

7. Zusammensetzung nach einem der vorhergehenden Ansprüche, **dadurch gekennzeichnet, dass** sie ein Gewichtsverhältnis von dem phenylierten Silikonöl zu dem Harz von 1 bis 5 und ebenfalls bevorzugt von 1,5 bis 4, bevorzugt von 2 bis 3,5 oder noch besser von 2,5 bis 3,5 aufweist.

8. Zusammensetzung nach einem der vorhergehenden Ansprüche, **dadurch gekennzeichnet, dass** es sich bei dem flüchtigen Öl um ein Kohlenwasserstofföl handelt, das aus Kohlenwasserstoffölen mit 8 bis 16 Kohlenstoffatomen, vorzugsweise flüchtigem Öl mit einem Flammpunkt von mehr als 80°C, wie Isohexadecan, ausgewählt ist.

9. Zusammensetzung nach einem der vorhergehenden Ansprüche, **dadurch gekennzeichnet, dass** sie einen Gehalt an flüchtigem Öl von 5 bis 50 New.-% und von 10 bis 35 Gew.-%, bezogen auf das Gesamtgewicht der Zusammensetzung, aufweist.

10. Zusammensetzung nach einem der vorhergehenden Ansprüche, **dadurch gekennzeichnet, dass** sie mindestens ein zusätzliches nicht-flüchtiges Kohlenwasserstofföl umfasst.

11. Zusammensetzung nach dem vorhergehenden Anspruch, **dadurch gekennzeichnet, dass** das zusätzliche nichtflüchtige Kohlenwasserstofföl aus Vinylpyrrolidon/1-Hexadecen-Copolymeren ausgewählt ist.

**12.** Zusammensetzung nach dem vorhergehenden Anspruch, **dadurch gekennzeichnet, dass** das zusätzliche nichtflüchtige Öl in einem Gehalt von 0, 1 bis 60 Ges.-%, insbesondere von 0,5 bis 50 Ges.-% und besonders von 1 bis 40 Gew.-%, bezogen auf das Gesamtgewicht der Zusammensetzung, vorhanden ist.

**13.** Zusammensetzung nach einem der vorhergehenden Ansprüche, **dadurch gekennzeichnet, dass** sie 0,1 bis 30 Ges.-% Wachs, bezogen auf das Gesamtgewicht der Zusammensetzung, umfasst.

**14.** Zusammensetzung nach einem der Ansprüche 1 bis 12, **dadurch gekennzeichnet, dass** sie frei von Wachs ist.

**15.** Zusammensetzung nach einem der vorhergehenden Ansprüche, **dadurch gekennzeichnet, dass** sie mindestens einen Füllstoff und/oder mindestens eine pastöse Fettsubstanz und/oder mindestens einen Farbstoff umfasst.

**16.** Zusammensetzung nach dem vorhergehenden Anspruch, **dadurch gekennzeichnet, dass** sie weniger als 3 Gew.-% oder besser weniger als 1 Ges.-% Wasser, bezogen auf das Gesamtgewicht der Zusammensetzung, umfasst oder sogar vollkommen wasserfrei ist.

**17.** Zusammensetzung nach einem der vorhergehenden Ansprüche, **dadurch gekennzeichnet, dass** die Zusammensetzung in fester Form vorliegt.

**18.** Verfahren zum Schminken oder zur Pflege der Haut und/oder der Lippen, bei dem man auf die Haut und/oder die Lippen eine Zusammensetzung nach einem der vorhergehenden Ansprüche aufbringt.

**19.** Verwendung einer Zusammensetzung nach einem der Ansprüche 1 bis 17 zum Erhalten von Schminken der Haut und/oder der Lippen, um einen Auftrag auf der Haut und/oder den Lippen zu erhalten, der zufriedenstellende Eigenschaften hinsichtlich Homogenität des Auftrags und/oder Brillanz und/oder Beständigkeit der Brillanz und/oder Beständigkeit der Farbe aufweist.

## Claims

**1.** Cosmetic makeup and/or care composition comprising at least one fatty phase comprising:

- at least one resin having a number-average molecular weight of less than or equal to 10 000 g/mol, chosen from indene hydrocarbon-based resins, and mixtures thereof,
- at least one volatile oil, and
- at least one phenyl silicone oil of formula (IV):

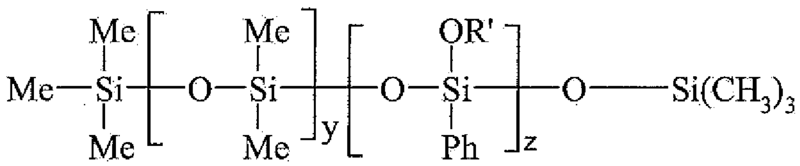

(IV)

in which Me represents a methyl group and Ph represents a phenyl group, OR' represents an $-OSiMe_3$ group and y varies between 1 and 1000, z varies between 1 and 1000.

**2.** Composition according to the preceding claim, **characterized in that** the indene hydrocarbon-based resin results from the polymerization of an indene monomer and of a monomer chosen from styrene, methylindene and methylstyrene, and mixtures thereof.

**3.** Composition according to either of the preceding claims, **characterized in that** the indene hydrocarbon-based resin is hydrogenated.

**4.** Composition according to any one of Claims 1 to 3, **characterized in that** the resin is an indene resin chosen from

hydrogenated indene/methylstyrene/styrene copolymers.

5. Composition according to any one of the preceding claims, **characterized in that** the resin is present in a content ranging from 0.1% to 30% by weight, relative to the total weight of the composition, preferably ranging from 0.3% to 20% by weight, more preferably ranging from 0.5% to 15% by weight.

6. Composition according to any one of the preceding claims, **characterized in that** the phenyl silicone oil is present in a content ranging from 0.5% to 85% by weight, particularly from 5% to 70% by weight, relative to the total weight of the composition.

7. Composition according to any one of the preceding claims, **characterized in that** it has a weight ratio of said phenyl silicone oil to said resin ranging from 1 to 5, and more preferably from 1.5 to 4, preferably from 2 to 3.5, or even better still from 2.5 to 3.5.

8. Composition according to any one of the preceding claims, **characterized in that** said volatile oil is a hydrocarbon-based oil chosen from hydrocarbon-based oils containing from 8 to 16 carbone atoms; preferably, the volatile oil has a flash point of greater than 80ºC, such as isohexadecane.

9. Composition according to any one of the preceding claims, **characterized in that** it has a volatile oil content ranging from 5% to 50% by weight, and ranging from 10% to 35% by weight, relative to the total weight of the composition.

10. Composition according to any one of the preceding claims, **characterized in that** it comprises at least one additional hydrocarbon-based oil which is non-volatile.

11. Composition according to the preceding claim, **characterized in that** the additional non-volatile hydrocarbon-based oil is chosen from vinylpyrrolidone/ 1-hexadecene copolymers.

12. Composition according to the preceding claim, **characterized in that** said additional non-volatile oil is present in a content ranging from 0.1% to 60% by weight, especially ranging from 0.5% to 50% by weight, and in particular ranging from 1% to 40% by weight, relative to the total weight of said composition.

13. Composition according to any one of the preceding claims, **characterized in that** it comprises from 0.1% to 30% by weight of wax, relative to the total weight of the composition.

14. Composition according to any one of Claims 1 to 12, **characterized in that** it is free of wax.

15. Composition according to any one of the preceding claims, **characterized in that** it comprises at least one filler and/or at least one pasty fatty substance and/or at least one dyestuff.

16. Composition according to the preceding claim, **characterized in that** it comprises less than 3%, or better still less than 1% of water by weight, relative to the total weight of the composition, or is even totally anhydrous.

17. Composition according to any one of the preceding claims, **characterized in that** said composition is in solid form.

18. Method for making up or caring for the skin and/or the lips, in which a composition as defined according to any one of the preceding claims is applied to the skin and/or the lips.

19. Use of a composition as defined according to any one of Claims 1 to 17, for making up the skin and/or the lips so as to obtain a deposit on the skin and/or the lips which has satisfactory properties in terms of homogeneity of the deposit and/or of gloss and/or of staying power of the gloss and/or of staying power of the colour.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1405625 A
- FR 2792190 A
- US 5874069 A
- US 5919441 A
- US 6051216 A
- US 5981680 A
- EP 542669 A
- EP 787730 A
- EP 787731 A
- WO 9608537 A


### Littérature non-brevet citée dans la description


- Handbook of Pressure Sensitive Adhesive. 1989, 609-619